# EUROPEAN PATENT SPECIFICATION

(11) **EP 1 154 793 B1**
(45) Date of publication and mention of the grant of the patent: **15.09.2010**
(21) Application number: 99930126.0
(22) Date of filing: 28.05.1999
(51) Int. Cl.: A61K 39/39, A61K 39/145, A61K 9/00, A61K 9/16, A61P 31/16

(54) **USE OF BIOADHESIVES AND ADJUVANTS FOR THE MUCOSAL DELIVERY OF ANTIGENS**
VERWENDUNG VON BIOADHAESIVEN UND ADJUVANTIEN FÜR DIE MUKOSALE ANWENDUNG VON ANTIGENEN
UTILISATION DE SYSTEMES BIOADHESIFS ET D'ADJUVANTS POUR L'ADMINISTRATION MUCOSALE D'ANTIGENES

(30) Priority: 26.02.1999 US 122172 P
(43) Date of publication of application: 21.11.2001
(73) Proprietor: Novartis Vaccines and Diagnostics, Inc., Emeryville, CA 94608 (US)
(72) Inventor: SINGH, Manmohan, Hercules, CA 94547 (US); O'HAGAN, Derek, Berkeley, CA 94008 (US)
(74) Representative: Hallybone, Huw George
(86) International application number: PCT/US1999/011906
(87) International publication number: WO 2000/050078

(56) References cited:
- EP-A- 0 304 786
- WO-A-94/20070
- WO-A-95/11700
- WO-A-95/17211
- WO-A-98/42375
- US-A- 5 707 644
- GIULIANI M M, ET AL.: "Mucosal Adjuvandicity and Immunogenicity of LTR72, a Novel Mutant of Escherichia coli Heat-labile Enterotoxin with Partial Knockout of ADP-ribosyltransferase Activity" JOURNAL OF EXPERIMENTAL MEDICINE, [Online] vol. 187, no. 7, 6 April 1998 (1998-04-06), pages 1123-1132, XP002118943 ISSN: 0022-1007 Retrieved from the Internet: <URL:http://intl.jem.org/content/vol187/is sue7/index.shtml> [retrieved on 1999-10-14]
- ONTSOUKA E C ET AL: "mRNA expression of motility-mediating receptors from the abomasum to the spiral colon of healthy cows and of cows suffering from left-sided abomasal displacement" POULTRY SCIENCE, vol. 86, no. Suppl. 1, 2007, page 469, & JOINT ANNUAL MEETING OF THE AMERICAN-DAIRY-SCIENCE-ASSOCIATION/POULTR Y-SCIENCE-ASSOCIATION-ASOCIACIO; SAN ANTONIO, TX, USA; JULY 08 -12, 2007 ISSN: 0032-5791

## Description

### FIELD OF THE INVENTION

The present invention relates generally to bioadhesive polymer systems. In particular, the invention relates to the use of bioadhesives and adjuvants for mucosal delivery of antigens.

### BACKGROUND OF THE INVENTION

Mucosal immunity provides an important defense mechanism against a wide variety of pathogens. In this regard, the mucosal surfaces of the gastrointestinal, respiratory and genitourinary tracts are continuously exposed to foreign antigens, including potentially infectious bacterial, viral, and sometimes parasitic organisms. Mucosal immune responses protect against such challenges and have distinct and specialized characteristics.

For example, the principal immunoglobulin produced by the mucosal immune system is secretory IgA. Specialized antigen uptake cells in the Peyer's Patches of the intestinal tract or nasopharyngeal lymphoid tissues, termed microfold or M cells, transport antigen to the underlying mucosal associated lymphoid tissues (MALT). In other areas of the mucosal epithelium, such as the pseudo-stratified airway epithelium, dendritic cells serve as antigen-presenting cells and migrate to local lymph nodes or MALT. Antigen processing and presentation occurs in the MALT, resulting in activation of antigen-specific IgA B cells. The subsequent trafficking and recirculation of the activated IgA-B cells to other components of the mucosal immune system, *e.g*., the respiratory, intestinal and genital tracts, provides for disseminated local mucosal IgA responses throughout the "Common Mucosal System." Thus, the mucosal immune system is uniquely suited to respond to the types of antigenic challenge encountered by mucosal surfaces, and can provide the most effective type of immune response against particular pathogens. Accordingly, antigen delivery mechanisms which target the mucosal immune system provide an attractive means for achieving immunity.

Attempts have been made to use bioadhesive polymers for the delivery of drugs. Bioadhesives are synthetic and naturally occurring materials able to adhere to biological substrates for extended time periods. Mucoadhesives are a subtype of bioadhesives that likely adhere to mucus. As a non-limiting example of mucoadhesives, Carbopol and polycarbophil, both synthetic cross-linked derivatives of poly(acrylic acid), display excellent adhesion properties *in vitro.* Another natural mucoadhesive is hyaluronic acid, also known as hyaluronan. Hyaluronic acid has been shown to be mucoadhesive, both *in vivo* and *in vitro.* See, *e.g.,* Cortivo et al., Biomaterials, 1991, 12:727-730; European Publication No. 517,565; WO 96/29998. Some bioadhesives can cause local irritation, however. Hence, few bioadhesive delivery systems are commercially available.

WO 94/20070 relates to polymeric mucoadhesives in the delivery of immunogens at mucosal surfaces, and to compositions comprising an antigen and a mucoadhesive, and optionally an adjuvant.

WO 95/17211 relates to a nontoxic mucosal adjuvant which may be admixed with further antigens to provide a vaccine administrable to mucosal surfaces, wherein the adjuvant is preferably a detoxified mutant of a bacterial ADP-ribosylating toxin.

WO 98/42375 relates to parenteral adjuvants comprising detoxified mutants of bacterial ADP-ribosylating toxins, particularly those from pertussis (PT), choleta (CT), and heat-labile E.coli (LT).

However, the use of bioadhesives, including mucoadhesives, and adjuvants to deliver vaccine "antigens", as defined herein, has not heretofore been described.

### SUMMARY OF THE INVENTION

Disclosed herein are compositions for use in an effective method for eliciting an immune response in a mammalian subject using mucosal immunization and bioadhesive delivery techniques.

The present invention is based on the discovery that the mucosal delivery of bioadhesives, including mucoadhesives and mucoadhesive derivatives, and an adjuvant, in combination with an antigen of interest, acts to enhance the immunogenicity of the antigen coadministered therewith. While not wishing to be bound by a particular theory, it is believed that the bioadhesive properties of the bioadhesives decrease the rate of clearance of the antigen and thus allow a longer contact time between the antigen and the absorbing membrane. Additionally, a transient widening may occur at the tight junctions between the cells of the mucosal epithelia allowing more efficient transport of the antigen of interest. The use of bioadhesives and adjuvants provides a safe and effective approach for enhancing the immunogenicity of a wide variety of antigens.

Accordingly, in one embodiment, the invention is directed to a composition as defined in claim 1, comprising (including) at least one bioadhesive, at least one adjuvant, and at least one selected antigen. The antigen and the adjuvant may each be, present in an amount of approximately 1% to about 40% (w/w) antigen or adjuvant to bioadhesive.P The bioadhesive is a mucoadhesive wherein the mucoadhesive is selected from cross-linked derivatives of poly(acrylic acid) .P In preferred embodiments of the present invention, the mucoadhesive is not hyaluronic acid.P The adjuvant is a detoxified mutant of a bacterial ADP-ribosylating toxin selected from LT-R72 or LT-K63.P In still further embodiments, the present invention is directed to methods of making pharmaceutical compositions which comprise (include) combining the above-described compositions of at least three components with pharmaceutically acceptable mucosal excipients. Further disclosed are methods of immunization comprising mucosally administering therapeutically effective amounts of the pharmaceutical compositions to a vertebrate subject. Further disclosed are methods of generating an immune response against a selected antigen comprising (including) mucosally administering therapeutically effective amounts of the pharmaceutical compositions to a vertebrate subject.

Further disclosed herein are directed to methods of immunization comprising mucosally administering therapeutically effective amounts of the pharmaceutical compositions comprising bioadhesives, adjuvants, antigens and excipients to a vertebrate subject, and methods of generating an immune response against a selected antigen comprising (including) mucosally administering therapeutically effective amounts of the pharmaceutical compositions comprising antigens, adjuvants, and bioadhesives to a vertebrate subject.

In still further embodiments, the present invention is directed to pharmaceutical compositions as defined in claim 9 which comprise (include) at least one antigen, at least one adjuvant, at least one bioadhesive, and at least one pharmaceutically acceptable mucosal excipients, for use in methods of immunization comprising mucosally administering therapeutically effective amounts of the pharmaceutical compositions to a vertebrate subject. Further disclosed are methods of generating an immune response against a selected antigen comprising (including) mucosally administering therapeutically effective amounts of the pharmaceutical compositions to a vertebrate subject.

These and other embodiments of the present invention will readily occur to those of ordinary skill in the art in view of the disclosure herein.

### BRIEF DESCRIPTION OF THE DRAWINGS

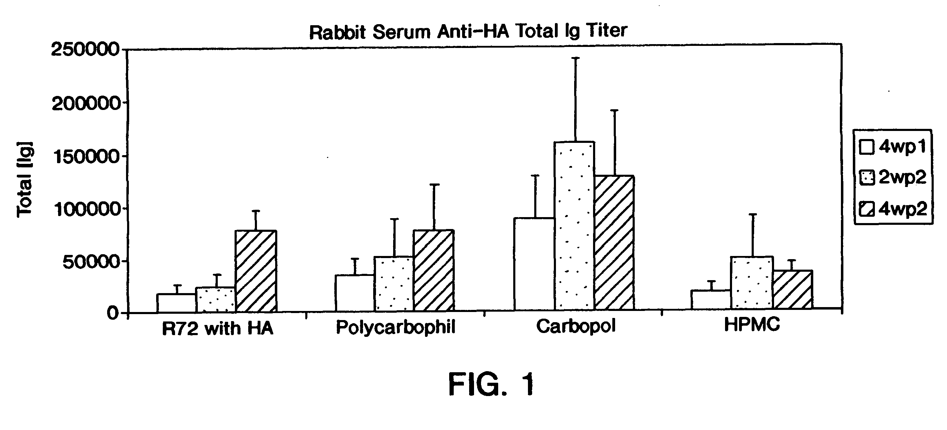

Figure 1 depicts the immunogenicity of the HA antigen in different bioadhesives in rabbits. Groups of rabbits that were administered antigen and adjuvant with carbopol or HPMC had higher titers than rabbits that were administered antigen and adjuvant alone.

### DETAILED DESCRIPTION OF THE INVENTION

The practice of the present invention will employ, unless otherwise indicated, conventional methods of chemistry, biochemistry, molecular biology, immunology and pharmacology, within the skill of the art. Such techniques are explained fully in the literature. See, *e.g.,* Remington's Pharmaceutical Sciences, 18th Edition (Easton, Pennsylvania: Mack Publishing Company, 1990); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell. Scientific Publications); and Sambrook, et al., Molecular Cloning: A Laboratory Manual (2nd Edition, 1989).

As used in this specification and the appended claims, the singular forms "a," "an" and "the" include plural references unless the content clearly dictates otherwise. Thus, for example, reference to "an antigen" includes a mixture of two or more such agents.

### I. Definitions

In describing the present invention, the following terms will be employed, and are intended to be defined as indicated below.

The term "mucoadhesive" refers to a bioadhesive agent that preferentially binds to mucus or mucosal cell surfaces. Examples of mucoadhesives known in the art include but are not limited to cross-linked derivatives of poly(acrylic acid), polyvinyl alcohol, hydroxypropyl methylcellulose (HPMC), polysaccharides, and carboxymethylcellulose. Carboxymethylcellulose, an exemplary bioadhesive, is shown below (structure 1).

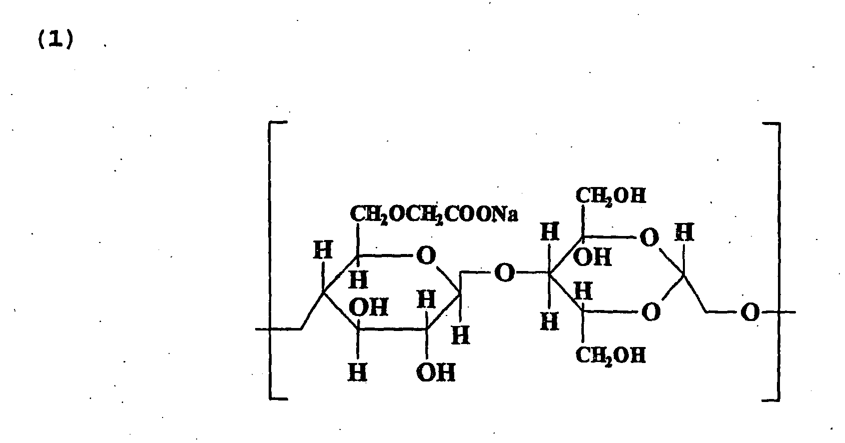

A "mucoadhesive derivative" is a molecule derived from a mucoadhesive and denotes any of various substances, known in the art, such as esterified mucoadhesives wherein approximately 75%-100% of the free carboxyl groups are esterified with an alkyl group. The term also includes "mixed" mucoadhesive esters, wherein carboxyl groups are esterified with more than one alkyl group. Such "mixed" esters are described more fully below. Furthermore, the term "mucoadhesive derivative" also refers to auto-crosslinked derivatives of mucoadhesives which include internal esters and in which about 0.5% to about 20% of the carboxyl groups of the mucoadhesives are crosslinked to hydroxyl groups of the same or different mucoadhesives.

The term "microsphere" as used herein, refers to a particle of about 100 nm to about 150 µm in diameter, more preferably about 200 nm to about 30 µm in diameter, and most preferably about 500 nm to about 10 µm in diameter. Microsphere size is readily determined by techniques well known in the art, such as photon correlation spectroscopy, laser diffractometry, and/or scanning electron microscopy. Microspheres for use herein will be formed from mucoadhesives and derivatives thereof, described in more detail, that are non-toxic and biodegradable.

The mucoadhesives of the present invention can also be used to form microspheres and/or microparticles, as described in PCT/US98/01738P The term "alkyl" as used herein refers to a branched or unbranched saturated hydrocarbon group of 1 to 24 carbon atoms, such as methyl, ethyl, n-propyl, isopropyl, n-butyl, isobutyl, t-butyl,- octyl, decyl, tetradecyl, hexadecyl, eicosyl, tetracosyl and the like, as well as cycloalkyl groups such as cyclopentyl, cyclohexyl, benzyl, and the like. The term "mucosal delivery" refers to the delivery of an antigen to a mucosal surface, including nasal, pulmonary, vaginal, rectal, urethral, and sublingual or buccal delivery.

The phrase "antigen" refers to any substance, including a protein or protein-polysaccharide, protein-lipopolysacchardie, polysaccharide, lipopolysacharide, polypeptides, DNA, RNA, and peptide nucleic acids (PNA), viral subunits, whole virus or whole bacteria which, when foreign to the blood stream of an animal, on gaining access to the tissue of such an animal, stimulates the formation of specific antibodies and reacts specifically *in vivo* or *in vitro* with a homologous antibody. Furthermore, an "antigen" can include modifications, such as deletions, additions and substitutions (generally conservative in nature), to the native sequence, so long as the modification maintains the ability to elicit an immunological response. These modifications can be deliberate, as, for example, through site-directed mutagenesis, or can be accidental, such as through mutations of hosts which produce the antigens. Moreover, an antigen, as used herein, stimulates the proliferation of T-lymphocytes, preferably Th1 lymphocytes, with receptors for the antigen and can react with the lymphocytes to initiate the series of responses designated cell-mediated immunity.

A hapten is within the scope of this definition of antigen. A hapten is that portion of an antigenic molecule or antigenic complex that determines its immunological specificity. Commonly, a hapten is a peptide or polysaccharide in naturally occurring antigens. In artificial antigens it can be a low molecular weight substance such as an arsanilic acid derivative. A hapten will react specifically *in vivo* or *in vitro* with homologous antibodies or T lymphocytes. Alternative descriptors are antigenic determinant, antigenic structural grouping, and haptenic grouping.

For purposes of the present invention, antigens can be derived from any of several known sources, including, without limitation, viruses, bacteria, parasites, and fungi. The term also intends any of the various tumor antigens.

As used herein, the term immunization refers to the process of generating an immunological response through the administration of an antigen to an individual.

An "immunological response" to an antigen or composition is the development in a subject of a humoral and/or a cellular immune response to molecules present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the intracellular destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells.

As used herein, an "immunogenic composition" or a vaccine is a composition that elicits an immunological response. Immunogenic compositions or vaccines that elicit a cellular immune response can serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen or immunogenic composition to stimulate a cell-mediated immunological response can be determined by a number of assays, such as by lymphoproliferation (lymphocyte activation) assays, CTL cytotoxic cell assays, or by assaying for T-lymphocytes specific for the antigen in a sensitized subject. Such assays are well known in the art. See, *e.g*., Erickson et al., J. Immunol., 1993, 151:4189-4199; Doe et al., Eur. J. Immunol., 1994, 24:2369-2376; and the examples below.

Thus, an immunological response as used herein can be one which stimulates the production of CTLs, and/or the production or activation of helper T-cells. The antigen of interest can also elicit an antibody-mediated immune response. Hence, an immunological response can include one or more of the following effects: the production of antibodies by B-cells and/or the activation of suppressor T-cells and/or γδ T-cells directed specifically to an antigen or antigens present in the composition or vaccine of interest. These responses can serve to neutralize infectivity, and/or mediate antibody-complement, or antibody dependent cell cytotoxicity (ADCC) to provide protection to an immunized host. Such responses can be determined using standard immunoassays and neutralization assays which are well known in the art.

An immunogenic composition which contains a selected antigen in combination with a bioadhesive and an adjuvant as described herein, displays "enhanced immunogenicity" when it possesses a greater capacity to elicit an immune response than the immune response elicited by an equivalent amount of the antigen and adjuvant without the bioadhesive. Thus, an immunogenic composition can display "enhanced immunogenicity" because the antigen is more readily absorbed by the vertebrate subject, or because the antigen is more strongly immunogenic or because a lower dose of antigen is necessary to achieve an immune response in the subject to which it is administered. Such enhanced immunogenicity can be determined by administering the bioadhesive/antigen/adjuvant composition, and antigen/adjuvant controls to animals and comparing antibody titers against the two using standard assays such as radioimmunoassay (RIA) and enzyme linked immunoassay (ELISA), both of which are well known in the art.

The terms "effective amount" or "pharmaceutically effective amount", as used herein, refer to a nontoxic but sufficient amount of the composition to provide the desired immunological response and corresponding therapeutic effect. As will be pointed out below, the exact amount required will vary from subject to subject, depending on the species, age, and general condition of the subject, the severity of the condition being treated, the particular antigen of interest, mode of administration, and the like. An appropriate "effective" amount in any individual case can be determined by one of ordinary skill in the art using routine experimentation.

As used herein, "treatment" refers to any of (i) the prevention of infection or reinfection, as in a traditional vaccine, (ii) the reduction or elimination of symptoms, and (iii) the substantial or complete elimination of the pathogen in question. Treatment can be effected prophylactically (prior to infection) or therapeutically (following infection). The term "treatment" also refers to enhancing the immunological response to an antigen.

As used herein, the term "enhancing" refers to an increase in the level of immunological response to an antigen and can include an increase in the response of the humoral and/or cellular branch of the immune system.

By "pharmaceutically acceptable" or "pharmacologically acceptable" is meant a material which is not biologically or otherwise undesirable, *i.e*., the material can be administered to an individual along with the microparticle formulations without causing any undesirable biological effects or interacting in a deleterious manner with any of the components of the composition in which it is contained.

By "vertebrate subject" is meant any member of the subphylum chordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term "vertebrate subject" does not denote a particular age. Thus, both adult and newborn subjects are intended to be covered. The system described above is intended for use in any of the above vertebrate species, since the immune systems of all of these vertebrates operate similarly.

### II. Modes of Carrying Out the Invention

Before describing the present invention in detail, it is to be understood that this invention is limited by the claims. . It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein. Bioadhesive-mediated delivery techniques to elicit an immune response against mucosally transmitted and systemic pathogens are disclosed. The system elicits a vigorous immune response, even when the antigen is by itself weakly immunogenic.

Attention has turned to the development of bioadhesives, especially mucoadhesives, as mucosal delivery systems. Such systems are based on naturally occurring substances, such as lectins and fimbrial proteins, and on artificial substances, such as derivatives of poly(acrylic acid), hydroxypropyl methylcellulose (HPMC), polyvinyl alcohols, and polysaccharides. These bioadhesives are likely to adhere to mucus,

The method disclosed herein provides for cell-mediated immunity, and/or humoral antibody responses. Accordingly, the methods of the present invention will find use with any antigen for which cellular and/or humoral immune responses are desired, derived from viral, pathogens that can induce antibodies, T-cell helper epitopes and T-cell cytotoxic epitopes. Such antigens include, but are not limited to, those encoded by human and animal viruses and can correspond to either structural or non-structural proteins.

For example, the composition of claim 1 will find use for stimulating an immune response against a wide variety of proteins from the herpesvirus family, including proteins derived from herpes simplex virus (HSV) types 1 and 2, such as HSV-1 and HSV-2 glycoproteins gB, gD and gH; antigens derived from varicella zoster virus (VZV), Epstein-Barr virus (EBV) and cytomegalovirus (CMV) including CMV gB and gH; and antigens derived from other human herpesviruses such as HHV6 and HHV7. (See, e.g. Chee et al., Cytomegaloviruses (J.K. McDougall, ed., Springer-Verlag 1990) pp. 125-169, for a review of the protein coding content of cytomegalovirus; McGeoch et al., J. Gen. Virol., 1988, 69:1531-1574, for a discussion of the various HSV-1 encoded proteins; U.S. Patent No. 5,171,568 for a discussion of HSV-1 and HSV-2 gB and gD proteins and the genes encoding therefor; Baer et al., Nature, 1984, 310:207-211, for the identification of protein coding sequences in an EBV genome; and Davison and Scott, J. Gen. Virol., 1986, 67:1759-1816, for a review of VSV.)

Antigens from the hepatitis family of viruses, including hepatitis A virus (HAV), hepatitis B virus (HBV), hepatitis C virus (HCV), the delta hepatitis virus (HDV), hepatitis E virus (HEV) and hepatitis G virus (HGV), can also be conveniently used in the techniques described herein. By way of example, the viral genomic sequence of HCV is known, as are methods for obtaining the sequence. See, e.g., International Publication Nos. WO 89/04669; WO 90/11089; and WO 90/14436. The HCV genome encodes several viral proteins, including E1 (also known as E) and E2 (also known as E2/NSI) and an N-terminal nucleocapsid protein (termed "core") (see, Houghton et al., Hepatology, 1991, 14:381-388, for a discussion of HCV proteins, including E1 and E2). Each of these proteins, as well as antigenic fragments thereof, will find use in the present methods. Similarly, the sequence for the δ-antigen from HDV is known (see, *e.g.,* U.S. Patent No. 5,378,814) and this antigen can also be conveniently used in the present methods. Additionally, antigens derived from HBV, such as the core antigen, the surface antigen, sAg, as well as the presurface sequences, pre-S1 and pre-S2 (formerly called pre-S), as well as combinations of the above, such as sAg/pre-S1, sAg/pre-S2, sAg/pre-S1/pre-S2, and pre-S1/pre-S2, will find use herein. See, *e.g*., "HBV Vaccines - from the laboratory to license: a case study" in Mackett, M. and Williamson, J.D., Human Vaccines and Vaccination, pp. 159-176, for a discussion of HBV structure; and U.S. Patent Nos. 4, 722, 840, 5,098,704, 5, 324, 513 ; Beames et al., J. Virol., 1995, 69:6833-6838, Birnbaum et al., J. Virol., 1990, 64: 3319-3330; and Zhou et al., J. Virol., 1991, 65 : 5457-5464.

Antigens derived from other viruses will also find use in the claimed methods, such as without limitation, proteins from members of the families Picornaviridae (*e.g*., polioviruses, etc.); Caliciviridae; Togaviridae (*e.g*., rubella virus, dengue virus, etc.); Flaviviridae; Coronaviridae; Reoviridae; Birnaviridae; Rhabodoviridae *(e.g.,* rabies virus, etc.); Filoviridae; Paramyxoviridae (*e.g*., mumps virus, measles virus, respiratory syncytial virus, etc.); Orthomyxoviridae (*e.g.,* influenza virus types A, B and C, etc.); Bunyaviridae; Arenaviridae; Retroviradae (*e.g.,* HTLV-I; HTLV-II; HIV-1 (also known as HTLV-ITI, LAV, ARV, hTLR, etc.)), including but not limited to antigens from the isolates HIV_{IIIb}, HIV_{SF2}, HIV_{LAV}, HIV_{LAI}, HIV_{MN}; HIV-1_{CM235}, HIV-1_{US4}; HIV-1 subtype (O) ; HIV-2; simian immunodeficiency virus (SIV) among others. Additionally, antigens can also be derived from human papillomavirus (HPV) and the tick-borne encephalitis viruses. See, *e.g*. Virology, 3rd Edition (W.K. Joklik ed. 1988); Fundamental Virology, 2nd Edition (B.N. Fields and D.M. Knipe, eds. 1991), for a description of these and other viruses.

More particularly, the gp120 envelope proteins from any of the above HIV isolates, including members of the various genetic subtypes of HIV, are known and reported (see, *e.g*., Myers et al., Los Alamos Database, Los Alamos National Laboratory, Los Alamos, New Mexico (1992); Myers et al., Human Retroviruses and Aids, 1990, Los Alamos, New Mexico: Los Alamos National Laboratory; and Modrow et al., J. Virol., 1987, 61:570-578, for a comparison of the envelope sequences of a variety of HIV isolates) and antigens derived from any of these isolates will find use in the present methods. Furthermore, the invention is equally applicable to other immunogenic proteins derived from any of the various HIV isolates, including any of the various envelope proteins such as gp160 and gp41, gag antigens such as p24gag and p55gag, as well as proteins derived from the pol region.

Disclosed herein are also numerous bacterial antigens, such as those derived from organisms that cause diphtheria, cholera, tuberculosis, tetanus, pertussis, meningitis, and other pathogenic states, including, without limitation; *Meningococcus* A, B and C, *Hemophilus influenza* type B (Hib), *Neisseria gonorrhoeae,* and *Helicobacter pylori.* Examples of parasitic antigens include those derived from organisms causing malaria and Lyme disease.

Furthermore, the methods described herein provide a means for treating a variety of malignant cancers. For example, the composition of the present invention can be used to mount both humoral and cell-mediated immune responses to particular proteins specific to the cancer in question, such as an activated oncogene, a fetal antigen, or an activation marker. Such tumor antigens include any of the various MAGEs (melanoma associated antigen E), including MAGE 1, 2, 3, 4, etc. (Boon, T. Scientific American (March 1993) : 82-89) ; any of the various tyrosinases; MART 1 (melanoma antigen recognized by T cells), mutant ras; mutant p53; p97 melanoma antigen; CEA (carcinoembryonic antigen), among others.

It is readily apparent that the subject invention can be used to prevent or treat a wide variety of diseases.

The present disclosure further provides methods for immunization, generating an immune response, and enhancing an immunological response comprising administering a pharmaceutical composition comprising at least one antigen, at least on adjuvant, at least one bioadhesive, and at least one pharmaceutically acceptable mucosal excipient.

Although the invention is broadly applicable to any of the above-mentioned pathogens, the invention is exemplified herein by reference to influenza virus. Specifically, the envelope glycoproteins HA and NA of influenza A are of particular interest for generating an immune response. Numerous HA subtypes of influenza A have been identified (Kawaoka et al., Virology, 1990, 179:759-767; Webster et al., "Antigenic variation among type A influenza viruses," p. 127-168. In: P. Palese and D.W. Kingsbury (ed.), Genetics of influenza viruses. Springer-Verlag, New York). Thus, proteins derived from any of these isolates can also be used in the immunization techniques described herein.

The selected antigen is combined with the bioadhesive and adjuvant for subsequent mucosal delivery. Useful bioadhesives in the methods described herein include, but are not limited to, cross-linked derivatives of poly(acrylic acid), polyvinyl alcohol, hydroxypropyl methylcellulose, polysaccharides, and carboxymethylcellulose. Many of these polymers are available in a variety of molecular weights, and the appropriate molecular weight for use with a given antigen is readily determined through routine experimentation by one of skill in the art.

The bioadhesives of the present invention can be provided as microspheres, either with adsorbed or physically incorporated (entrapped) antigen, using any of several techniques, well known in the art. See, *e.g*., Benedetti et al., J. Controlled Rel., 1990, 13:33-41; Ghezzo et al., Int. J. Pharm., 1992, 87:21-29; Illum et al., J. Controlled Rel., 1994, 29:133-141; European Publication No. 517,565; PCT US98/01738.

The production of microspheres by mixing the discontinuous phase with the continuos phase is well known to those of skill in the art. See, *e.g.,* U.S. Patent No. 3,891,570 and Benedetti et al., J. Controlled Rel., 1990, 13:33-41. Solvent extraction from microsphere preparations is also well known to those of skill in the art. For a further description of the solvent extraction technique, see, *e.g.,* Illum et al., J. Controlled Rel., 1994, 29:133-141; and Ghezzo et al., Int. J. Pharm., 1992, 87:21-29; and European Publication No. 517,565.

Alternatively, microspheres can also be formed using spray-drying, as described in, *e.g*., Kyyronen et al., In t. J. Pharm., 1992, 80:161-169; Ghezzo et al., Int. J. Pharm., 1992, 87:21-29; and Masters, K. (1976) Spray Drying 2nd Ed. Wiley, New York. Especially small microspheres, termed "nanospheres" can be produced using supercritical antisolvents (SAS), as described in International Publication No. WO 96/29998.

The rate of release of the antigen from the bioadhesive compositions can be modified depending on the method used to associate the antigen with the microspheres. For example, if the antigen is physically dispersed in the polymer matrix, release is controlled largely by the diffusion rate of the antigen through the polymer network. Furthermore, if solvents are extracted rather than evaporated, microspheres include more porous surfaces which result in more rapid release of the entrapped antigen.

Furthermore, esterification of carboxyl groups reduces the bioadhesiveness of bioadhesives due to the reduced tendency for esters to form hydrogen bonds with the biological substrate. Additionally, the hydrophobicity of the microspheres, imparted by differing esters and degrees of crosslinking, will affect the amount of bioadhesion since mucosal tissue appears to display appreciable hydrophobicity which can have important implications for bioadhesion. Thus, for example, a higher degree of esterification generally gives rise to slower and reduced release of the entrapped protein but produces a microsphere with enhanced bioadhesive properties.

Additionally, biological factors such as ciliary beat frequency, as well as physical factors such as particle size, density and degree of clumping, and water solubility of the antigen, will influence the degree of bioadhesion and bioerosion See, *e.g*., Pritchard et al., Int. J. Pharm., 1996, 129:137-145.

Moreover, mixtures of microspheres with varying esters, varying amounts of esterification, as well as varying degrees of crosslinking, will find use in the formulations in order to achieve the desired bioadhesion and release kinetics for a given antigen and to provide for both a primary and secondary immune response.

Once formed, the adhesiveness of a particular bioadhesive/antigen/adjuvant combination can be determined using any number of methods, well known in the art, in order to assess whether a particular formulation has appropriate bioadhesive properties. For example, *in vitro* detachment weight studies can be conducted which are based on surface tension measurements. See, *e.g*., Smart et al., J. Pharm. Pharmacol., 1984, 36:295-299. Briefly, test microspheres are applied to a biological substrate, such as epithelial tissue, and detachment weight studies conducted using an apparatus that determines the weight required to detach two tissue sections from the test bioadhesive which is sandwiched between them. See, *e.g.,* Pritchard et al., Int. J. Pharm., 1996, 129:137-145. Alternatively, mucociliary transport rate can be used as a determinant of adhesiveness since the greater the adhesiveness of the test substance, the slower the transport rate. Such studies can be conducted by, *e.g.,* monitoring the movement of bioadhesives along part of an excised upper palate from the frog *(Rana pipiens),* as described in Pritchard *et al., supra.*

Similarly, the rate of bioerosion of the microspheres can be determined using standard techniques, well known in the art, such as by *in vitro* release profiles, to determine whether the bioadhesive/adjuvant/antigen formulation in question provides an adequate amount of antigen to the immune system for the given disease. For example, dissolution tests can be performed by *e.g*., dispersing microspheres in an appropriate buffer such as a phosphate buffer or BSA, with continuous stirring. Samples of the solution are removed at fixed time intervals and assayed for the antigen of interest using, *e.g*., ELISAs or any other appropriate assay. See, Ghezzo et al., Int. J. Pharm., 1992, 87:21-29.

Particle size can be determined by, *e.g*., laser light scattering, using for example, a spectrometer incorporating a helium-neon laser. Generally, particle size is determined at room temperature and involves multiple analyses of the sample in question (*e.g*., 5-10 times) to yield an average value for the particle diameter. Particle size is also readily determined using scanning electron microscopy (SEM). In order to do so, dry microspheres are sputter-coated with a gold/palladium mixture to a thickness of approximately 100 Angstroms, then examined using a scanning electron microscope.

If the antigen is provided in a microsphere, the antigen content is generally determined so that an appropriate amount of the microspheres can be delivered to the subject in order to elicit an adequate immune response. Antigen content can be determined according to methods known in the art, such as by disrupting the microspheres and extracting any entrapped antigen. For example, microspheres can be dissolved in a solvent such as DMSO or dispersed in, *e.g*., 0.1 M NaOH containing 5% (w/v) SDS. The sample is agitated, optionally centrifuged, and the supernatant assayed for the antigen of interest using an appropriate assay. See, *e.g*., Benedetti et al., J. Controlled Rel., 1990, 13:33-41; and O'Hagan et al., Int. J. Pharm., 1994, 103:37-45.

For example, the antigen is generally incubated with the bioadhesive in an amount that represents approximately 1% to about 40% (w/w) antigen to bioadhesive, more preferably about 0.5% to about 25% (w/w) antigen, and even more preferably about 1% to about 10% (w/w) antigen. The percentage of antigen will depend on the desired dose and the condition being treated, as discussed in more detail below. The adjuvant is generally incubated with the bioadhesive in an amount that represents approximately .1% to about 40% (w/w) adjuvant to bioadhesive, more preferably about 0.5% to about 25% (w/w) adjuvant, and even more preferably about 1% to about 10% (w/w) adjuvant. The percentage of adjuvant will depend on the desired dose and the condition being treated, as discussed in more detail below. Incubation of antigen and adjuvant with polymer will proceed for approximately 0 hours to 48 hours or more, preferably about 0 hours to about 24 hours, more preferably about 1 hour to about 10 hours, and most preferably about 2 hours to about 4 hours. Following incubation, the suspension can be lyophilized and the dried composition suspended in an appropriate vehicle prior to immunization.

Once the antigen, adjuvant, and bioadhesive are made, as described above, compositions are formulated for subsequent mucosal delivery. The compositions will generally include one or more "pharmaceutically acceptable excipients or vehicles" appropriate for mucosal delivery, such as water, saline, glycerol, polyethyleneglycol, hyaluronic acid, ethanol, etc. Additionally, auxiliary substances, such as wetting or emulsifying agents, pH buffering substances, and the like, can be present in such vehicles.

For example, intranasal formulations will usually include vehicles that neither cause irritation to the nasal mucosa nor significantly disturb ciliary function. Diluents such as water, aqueous saline or other known substances can be employed with the subject invention. The nasal formulations can also contain preservatives such as, but not limited to, chlorobutanol and benzalkonium chloride. A surfactant can be present to enhance absorption of the subject proteins by the nasal mucosa. Intranasal formulations can be delivered, without limitation, in the form of inhalants or sprays.

For rectal and urethral suppositories, the vehicle composition will include traditional binders and carriers, such as, cocoa butter (theobroma oil) or other triglycerides, vegetable oils modified by esterification, hydrogenation and/or fractionation, glycerinated gelatin, polyalkaline glycols, mixtures of polyethylene glycols of various molecular weights and fatty acid esters of polyethylene glycol.

For vaginal delivery, the formulations of the present invention can be incorporated in pessary bases, such as those including mixtures of polyethylene triglycerides, or suspended in oils such as corn oil or sesame oil, optionally containing colloidal silica. See, *e.g*., Richardson et al., Int. J. Pharm., 1995, 115:9-15.

For a further discussion of appropriate vehicles to use for particular modes of delivery, see, *e.g.,* Remington: The Science and Practice of Pharmacy, Mack Publishing Company, Easton, Pennsylvania, 19th edition, 1995. One of skill in the art can readily determine the proper vehicle to use for the particular antigen and site of delivery.

The adjuvants used in this formulation can enhance the effectiveness of the pharmaceutical compositions. The adjuvants are preferably administered concurrently with the bioadhesive formulations of the present invention, *e.g*., in the same composition, but may also be administered in separate formulations. An additional adjuvant can be administered prior or subsequent to the bioadhesive formulations of the present invention. Adjuvants used herein in the formulation or as additional adjuvants include, but are not limited to: (1) aluminum salts (alum), such as aluminum hydroxide, aluminum phosphate, aluminum sulfate, etc.; (2) oil-in-water emulsion formulations (with or without other specific immunostimulating agents such as muramyl peptides (see below) or bacterial cell wall components), such as for example (a) MF59 (International Publication No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi^{™} adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox^{™}) ; (3) saponin adjuvants, such as Stimulon^{™} (Cambridge Bioscience, Worcester, MA) can be used or particle generated therefrom such as ISCOMs (immunostimulating complexes); (4) Complete Freunds Adjuvant (CFA) and Incomplete Freunds Adjuvant (IFA); (5) cytokines, such as interleukins (IL-1, IL-2, etc.), macrophage colony stimulating factor (M-CSF), tumor necrosis factor (TNF), etc.; (6) detoxified mutants of a bacterial ADP-ribosylating toxin such as a cholera toxin (CT), a pertussis toxin (PT), or an E. *coli* heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, *e.g*., International Publication Nos. W093/13202 and W092/19265); and (7) other substances that act as immunostimulating agents to enhance the effectiveness of the composition.
Muramyl peptides include, but are not limited to, N-acetyl-muramyl-L-threonyl-D-isoglutamine (thr-MDP), N-acteyl-normuramyl-L-alanyl-D-isogluatme (nor-MDP), and N-acetylmuramyl-L-alanyl-D-isogluatminyl-L-alanine-2-(1'-2'-dipalmitoyl-*sn*-glycero-3-huydroxyphosphoryloxy)-ethylamine (MTP-PE).

The various components of the composition can be present in a wide range of ratios. For example, the antigen and bioadhesive components are typically used in a volume range of 1:10 to 10:1, preferably 1:50 to 50:1, and most preferably 1:100. The adjuvant and bioadhesive components are typically used in a volume range of 1:10 to 10:1, preferably 1:50 to 50:1, and most preferably 1:100. The antigen and adjuvant components are typically used in a volume range of 1:10 to 10:1, preferably 1:5 to 5:1, more preferably from about 1:2 to 2:1, and most preferably about 1:1. However, other ratios can be more appropriate for specific purposes, such as when a particular antigen is both difficult to incorporate into a bioadhesive composition and has a low immunogenicity, in which case a higher relative amount of the antigen component is required.

The compositions will comprise a "therapeutically effective amount" of the antigen of interest. That is, an amount of antigen will be included in the compositions which will cause the subject to produce a sufficient immunological response in order to prevent, reduce or eliminate symptoms. The exact amount necessary will vary, depending on the subject being treated; the age and general condition of the subject to be treated; the capacity of the subject's immune system to synthesize antibodies; the degree of protection desired; the severity of the condition being treated; the particular antigen selected and its mode of administration, among other factors. An appropriate effective amount can be readily determined by one of skill in the art. Thus, a "therapeutically effective amount" will fall in a relatively broad range that can be determined through routine trials. For example, for purposes of the present invention, an effective dose will typically range from about 1 µg to about 100 mg, more preferably from about 5 µg to about 1 mg, and most preferably about 10 µg to about 500 µg of the antigen delivered per dose.

Once formulated, the compositions of the invention can be administered mucosally, using standard techniques. See, *e.g.,* Remington: The Science and Practice of Pharmacy, Mack Publishing Company, Easton, Pennsylvania, 19th edition, 1995**,** for mucosal delivery techniques, including intranasal, pulmonary, vaginal and rectal techniques, as well as European Publication No. 517,565 and Illum et al., J. Controlled Rel., 1994, 29:133-141, for techniques of intranasal administration. Alternatively, the compositions of the present invention may be administered dermally or transdermally, using standard techniques. See, *e.g.,* Remington: The Science and Practice of Pharmacy, Mack Publishing Company, Easton, Pennsylvania, 19th edition, 1995.

Dosage treatment can be a single dose schedule or a multiple dose schedule. A multiple dose schedule is one in which a primary course of vaccination can be with 1-10 separate doses, followed by other doses given at subsequent time intervals, chosen to maintain and/or reinforce the immune response, for example at 1-4 months for a second dose, and if needed, a subsequent dose(s) after several months. The boost can be with the same formulation given for the primary immune response, or can be with a different formulation that contains the antigen. The dosage regimen will also, at least in part, be determined by the need of the subject and be dependent on the judgment of the practitioner. Furthermore, if prevention of disease is desired, the bioadhesive compositions are generally administered prior to primary infection with the pathogen of interest. If treatment is desired, *e.g.,* the reduction of symptoms or recurrences, the bioadhesive compositions are generally administered subsequent to primary infection.

The formulations can be tested *in vivo* in a number of animal models developed for the study of mucosal, dermal, or transdermal delivery. As is readily apparent, the compositions of the present invention are useful for treating and/or preventing a wide variety of diseases and infections caused by viruses, bacteria, parasites, and fungi, as well as for eliciting an immune response against a variety of antigens. Not only can the compositions be used therapeutically or prophylactically, as described above, the compositions can also be used in order to prepare antibodies, both polyclonal and monoclonal, for, *e.g*., diagnostic purposes, as well as for immunopurification of the antigen of interest. If polyclonal antibodies are desired, a selected mammal, (*e.g*., mouse, rabbit, goat, horse, etc.) is immunized with the compositions of the present invention. The animal is usually boosted 2-6 weeks later with one or more - administrations of the antigen. Polyclonal antisera is then obtained from the immunized animal and treated according to known procedures. See, *e.g*., Jurgens et al., J. Chrom. 1985, 348:363-370.

Monoclonal antibodies are generally prepared using the method of Kohler and Milstein, Nature, 1975, 256:495-96, or a modification thereof. Typically, a mouse or rat is immunized as described above. However, rather than bleeding the animal to extract serum, the spleen (and optionally several large lymph nodes) is removed and dissociated into single cells. If desired, the spleen cells can be screened (after removal of nonspecifically adherent cells) by applying a cell suspension to a plate or well coated with the protein antigen. B cells, expressing membrane-bound immunoglobulin specific for the antigen, will bind to the plate, and are not rinsed away with the rest of the suspension. Resulting B cells, or all dissociated spleen cells, are then induced to fuse with myeloma cells to form hybridomas, and are cultured in a selective medium (*e.g.,* hypoxanthine, aminopterin, thymidine medium, "HAT"). The resulting hybridomas are plated by limiting dilution, and are assayed for the production of antibodies which bind specifically to the immunizing antigen (and which do not bind to unrelated antigens). The selected monoclonal antibody-secreting hybridomas are then cultured either *in vitro* (*e.g.,* in tissue culture bottles or hollow fiber reactors), or *in vivo* (as ascites in mice). See, *e.g*., M. Schreier et al., Hybridoma Techniques 1980; Hammerling et al., Monoclonal Antibodies and T-cell Hybridomas, 1981; Kennett et al., Monoclonal Antibodies, 1980; see also U.S. Patent Nos. 4,341,761; 4,399,121; 4,427,783; 4,444,887; 4,452,570; 4,466,917; 4,472,500, 4,491,632; and 4,493,890. Panels of monoclonal antibodies produced against the polypeptide of interest can be screened for various properties; *i.e*., for isotype, epitope, affinity, etc.

### III. Experimental

Below is an example of specific embodiments for carrying out the present invention. The example is offered for illustrative purposes only, and is not intended to limit the scope of the present invention in any way.

Efforts have been made to ensure accuracy with respect to numbers used (*e.g*., amounts, temperatures, etc.), but some experimental error and deviation should, of course, be allowed for.

### Example 1

### Comparison of Different Bioadhesives

The immunogenicity of the HA antigen in different bioadhesives was compared in rabbits (New Zealand strain).

In order to achieve a dose of 25 µg influenza antigen H3N2 ("HA") (Chiron Vaccines, Sienna, Italy) and 25 µg LT-R72 (International Publication No. WO 93/13202), a 1:1:100 ratio (antigen:adjuvant:bioadhesive) was targeted. The HA antigen was supplied in solution at a concentration of about 1 mg/ml. Adjuvants were also supplied in solution at concentrations between about 1-3 mg/ml. The bioadhesives hydroxypropyl methylcellulose (HPMC), Carbopol, and polycarbophil (B.F. Goodrich, Cleveland, Ohio) were prepared by suspending the product as received in PBS to obtain a 0.5% w/w gel. To facilitate formation of bioadhesives, the carbopol solution was neutralized with 0.2N NaOH to pH 7.2. The three components were mixed by stirring.

Thirty rabbits were divided into four groups, as shown in Table 1. In order to achieve the proper dose, all groups were administered the test compositions intranasally using a gauge 16 Teflon catheter.

Rabbits were administered antigen and adjuvant with or without bioadhesives in PBS in a total volume of 250 µl per animal. Formulations were administered within 60 minutes of preparation.

Rabbits received a booster at day 28. Sera was collected and assayed for anti-HA serum IgG levels using an ELISA. Sera was collected in 5 ml aliquots at 28 days post injection immediately preceding booster (4wp1), 42 days post injection (2 weeks post booster; 2wp2), and 56 days post injection (4 weeks post booster; 4wp2).

As can be seen in Table 1 and Figure 1, groups of rabbits that were administered antigen and adjuvant with carbopol or HPMC had higher titers than rabbits that were administered antigen and adjuvant alone.

| **Table 1** | | | | |
|---|---|---|---|---|
| Group | Formulation | Serum Anti-HA ELISA Titers (28 days) | Serum Anti-HA ELISA Titers (42 days) | Serum Anti-HA ELISA Titers (56 days) |
| 1 | LT-R72 (25 µg) + HA (25 µg) | 17248 +/- 8562 | 23350 +/- 12188 | 77463 +/- 19049 |
| 2 | LT-R72 (25 µg) + HA (25 µg) + polycarbophil (0.5% w/w) | 34448 +/- 16106 | 51851 +/- 36537 | 76876 +/- 43987 |
| 3 | LT-R72 (25 µg) + HA (25 µg) + carbopol (0.5% w/w) | 87951 +/- 40543 | 160255 +/- 78956 | 127882 +/- 61443 |
| 4 | LT-R72 (25 µg) + HA (25 µg) + HPMC (0.5% w/w) | 17556 +/- 9106 | 49985 +/- 40641 | 36052 +/- 10271 |

Accordingly, the use of bioadhesives and adjuvants to deliver antigens is described. Preferred embodiments of the subject invention have been described in some detail.

## Claims

1. A composition comprising at least one bioadhesive, at least one adjuvant and at least one viral antigen, wherein the at least one adjuvant comprises a LT-R72 or LT-K63 detoxified mutant of a bacterial
ADP-ribosylating toxin, wherein the at least one bioadhesive is a mucoadhesive selected from crosslinked derivatives of poly(acrylic acid)

2. The composition of claim 1, wherein the antigen is present at about 0.1 % to 40% (w/w) antigen to bioadhesive and the adjuvant is present at about 0.1% to 40% (w/w) adjuvant to bioadhesive.

3. The composition of claim 1, wherein the crosslinked derivative of poly(acrylic acid) is carbopol or polycarbophil.

4. The composition of any preceding claim, wherein the at least one adjuvant further comprises an adjuvant selected from the group consisting of alum, oil-in-water emulsion formulations, and muramyl peptides.

5. The composition of any preceding claim wherein the viral antigen is an influenza antigen.

6. The composition of any preceding claim, wherein the bioadhesive is provided as a microsphere.

7. The composition of claim 6, wherein the antigen is encapsulated within the microsphere.

8. The composition of claim 6, wherein the antigen is adsorbed to the microsphere.

9. A pharmaceutical composition comprising the composition of any preceding claim and at least once pharmaceutically acceptable mucosal excipient.

10. A method of making a pharmaceutical composition comprising combining the composition of any one of claims 1 to 8 and at least one pharmaceutically acceptable mucosal excipient.

11. The composition of any one of claims 1 to 8, for use in medicine.

12. The use of at least one bioadhesive, at least one adjuvant, and at least one viral antigen, in the manufacture of a medicament for immunising a vertebrate subject, wherein the at least one adjuvant comprises a LT-R72 or LT-K63 detoxified mutant of a bacterial ADP-ribosylating toxin, wherein the at least one bioadhesive is a mucoadhesive selected from crosslinked derivatives of poly(acrylic acid).

## Patentansprüche

1. Zusammensetzung, die mindestens ein Bioadhäsiv, mindestens ein Adjuvans und mindestens ein virales Antigen umfasst, wobei das mindestens eine Adjuvans eine detoxifizierte LT-R72- oder LT-K63-Mutante eines bakteriellen ADP-ribosylierenden Toxins umfasst, und wobei das mindestens eine Bioadhäsiv ein Mukoadhäsiv ist, welches ausgewählt ist aus quervernetzten Derivaten von Poly(acrylsäure).

2. Zusammensetzung nach Anspruch 1, wobei das Antigen bei etwa 0,1% bis 40% (Gew./Gew.) Antigen zu Bioadhäsiv vorhanden ist, und wobei das Adjuvans bei etwa 0,1% bis 40% (Gew./Gew.) Adjuvans zu Bioadhäsiv vorhanden ist.

3. Zusammensetzung nach Anspruch 1, wobei das quervernetzte Derivat der Poly(acrylsäure) Carbopol oder Polycarbophil ist.

4. Zusammensetzung nach einem der vorhergehenden Ansprüche, wobei das mindestens eine Adjuvans ferner ein Adjuvans umfasst, welches ausgewählt ist aus der Gruppe bestehend aus Alum, Öl-in-Wasser-Emulsions-Formulierungen und Muramylpeptiden.

5. Zusammensetzung nach einem der vorhergehenden Ansprüche, wobei das virale Antigen ein Influenza-Antigen ist.

6. Zusammensetzung nach einem der vorhergehenden Ansprüche, wobei das Bioadhäsiv als Mikrosphäre bereitgestellt wird.

7. Zusammensetzung nach Anspruch 6, wobei das Antigen in der Mikrosphäre eingekapselt ist.

8. Zusammensetzung nach Anspruch 6, wobei das Antigen an die Mikrosphäre adsorbiert ist.

9. Pharmazeutische Zusammensetzung, welche die Zusammensetzung nach einem der vorhergehenden Ansprüche umfasst sowie mindestens einen pharmazeutisch akzeptablen mukosalen Hilfsstoff.

10. Verfahren zur Herstellung einer pharmazeutischen Zusammensetzung, bei dem man die Zusammensetzung nach einem der Ansprüche 1 bis 8 mit mindestens einem pharmazeutisch akzeptablen mukosalen Hilfsstoff kombiniert.

11. Zusammensetzung nach einem der Ansprüche 1 bis 8 zur Verwendung in der Medizin.

12. Verwendung von mindestens einem Bioadhäsiv, mindestens einem Adjuvans und mindestens einem viralen Antigen bei der Herstellung eines Medikament zur Immunisierung eines Vertebraten-Subjekts, wobei das mindestens eine Adjuvans eine detoxifizierte LT-R72- oder LT-K63-Mutante eines bakteriellen ADP-ribosylierenden Toxins umfasst, und wobei das mindestens eine Bioadhäsiv ein Mukoadhäsiv ist, welches ausgewählt ist aus quervernetzten Derivaten von Poly(acrylsäure).

## Revendications

1. Composition comprenant au moins un bioadhésif, au moins un adjuvant et au moins un antigène viral, dans laquelle le au moins un adjuvant comprend un mutant détoxifié LT-R72 ou LT-K63 d'une toxine ADP-ribosylante bactérienne, dans laquelle le au moins un bioadhésif est un mucoadhésif choisi parmi les dérivés réticulés de poly(acide acrylique).

2. Composition selon la revendication 1, dans laquelle l'antigène est présent en une quantité d'environ 0,1% à 40% (p/p) d'antigène par rapport au bioadhésif et l'adjuvant est présent en une quantité d'environ 0,1% à 40% (p/p) d'adjuvant par rapport au bioadhésif.

3. Composition selon la revendication 1, dans laquelle le dérivé réticulé de poly(acide acrylique) est du carbopol ou du polycarbophil.

4. Composition selon l'une quelconque des revendications précédentes, dans laquelle le au moins un adjuvant comprend en outre un adjuvant choisi dans le groupe constitué par l'alun, les formulations sous forme d'émulsions d'huile dans l'eau et les peptides de muramyle.

5. Composition selon l'une quelconque des revendications précédentes, dans laquelle l'antigène viral est un antigène de la grippe.

6. Composition selon l'une quelconque des revendications précédentes, dans laquelle le bioadhésif se présente sous la forme d'une microsphère.

7. Composition selon la revendication 6, dans laquelle l'antigène est encapsulé dans la microsphère.

8. Composition selon la revendication 6, dans laquelle l'antigène est adsorbé sur la microsphère.

9. Composition pharmaceutique comprenant la composition selon l'une quelconque des revendications précédentes et au moins un excipient mucosal acceptable sur le plan pharmaceutique.

10. Procédé de fabrication d'une composition pharmaceutique comprenant la combinaison de la composition selon l'une quelconque des revendications 1 à 8 et d'au moins un excipient mucosal acceptable sur le plan pharmaceutique.

11. Composition selon l'une quelconque des revendications 1 à 8, destinée à l'utilisation en médecine.

12. Utilisation d'au moins un bioadhésif, d'au moins un adjuvant et d'au moins un antigène viral dans la fabrication d'un médicament pour immuniser un sujet vertébré, dans laquelle le au moins un adjuvant comprend un mutant détoxifié LT-R72 ou LT-K63 d'une toxine ADP-ribosylante bactérienne, dans laquelle le au moins un bioadhésif est un mucoadhésif choisi parmi les dérivés réticulés de poly(acide acrylique).
